Europäisches Patentamt

European Patent Office

Office européen des brevets

(19)

(11) Numéro de publication : **0 099 289**
**B1**

# FASCICULE DE BREVET EUROPÉEN

(12)

(45) Date de publication du fascicule du brevet :
16.04.86

(51) Int. Cl.⁴ : **A 61 B 17/60**

(21) Numéro de dépôt : **83401391.4**

(22) Date de dépôt : **06.07.83**

(54) **Dispositif de fixation pour immobilisation des os longs à fracture ouverte.**

(30) Priorité : 09.07.82 FR 8212130

(43) Date de publication de la demande :
25.01.84 Bulletin 84/04

(45) Mention de la délivrance du brevet :
16.04.86 Bulletin 86/16

(84) Etats contractants désignés :
**AT BE CH DE GB IT LI NL SE**

(56) Documents cités :
**EP-A- 0 024 256**
**FR-A- 2 129 735**
**US-A- 2 048 832**
**US-A- 2 391 537**

(73) Titulaire : **ECOLE NATIONALE SUPERIEURE D'ARTS
ET METIERS CENTRE DE PARIS
151, Boulevard de l'Hôpital
F-75640 Paris Cedex 13 (FR)**

(72) Inventeur : **Lavaste, François
151, Boulevard de l'Hôpital
F-75640 Paris Cedex 13 (FR)**

(74) Mandataire : **Chevallier, Robert Marie Georges
Cabinet BOETTCHER 23, rue La Boétie
F-75008 Paris (FR)**

Jouve, 18, rue St-Denis, 75001 Paris, France

## Description

Dispositif de fixation pour immobilisation des os longs à fracture ouverte.

L'invention a pour objet un dispositif de fixation, que l'on appelle souvent plus simplement fixateur externe en chirurgie orthopédique, qui sert à tenir en position correcte pendant la durée nécessaire à leur réparation naturelle les parties des os longs qui présentent une fracture ouverte. On entend ici par os allongés principalement le fémur et le tibia. La durée de la réparation naturelle est de l'ordre de 8 à 10 mois. Il est donc important que les os soient tenus rigidement pendant toute cette durée dans la position qui leur est donnée après réduction de la fracture.

On utilise couramment à cet effet le fixateur d'Hoffmann le plus répandu, et le fixateur de Judet. Tous deux comprennent des broches qui traversent la chair, qui sont vissées à l'os à une extrémité et qui sont tenues solidement en position par leur autre extrémité à l'extérieur du membre fracturé. Dans le fixateur d'Hoffmann les broches transfixiantes sont réunies par des plaquettes qui sont elles-mêmes reliées par des tiges rigides. Dans un fixateur de Judet, les broches sont placées dans deux plans faisant un V à 110° et leurs extrémités libres sont reliées dans chaque plan par un profilé rigide en U.

Il existe aussi des fixateurs plus complexes qui combinent les moyens des fixateurs d'Hoffmann et de Judet en prévoyant deux plans de transfixion perpendiculaires ou même plus de deux plans de transfixion.

On connaît encore par le document FR-A-2 129 735 un dispositif de fixation pour fracture de diaphyse comprenant une barre qui est pourvue à une partie extrême de moyens de fixation à une première fraction d'un os brisé et qui porte sur sa partie extrême opposée une douille susceptible de coulisser ; cette douille est pourvue de moyens de fixation à une seconde fraction du même os ; elle est tenue en position sur la première barre à l'aide d'écrous qui se vissent sur cette dernière et qui agissent chacun sur une extrémité d'un empilement de rondelles élastiques dont l'autre extrémité est en butée contre une extrémité correspondante de ladite douille. Il existe donc entre la barre et la douille une liaison élastique mais à une valeur relativement élevée de la force de déformation élastique de sorte que, dans ce document, les moyens de fixation à l'os comprennent des fils élastiques qui traversent l'os et qui assurent un effet élastique à une valeur moins élevée de la force.

Or, s'il est impératif que le fixateur conserve toujours une rigidité complète à la flexion afin que les os restent bien dans l'alignement qui leur a été donné, il est préférable que le fixateur puisse être raccourci en longueur et qu'il permette un certain effet progressif de compression — sans aucune possibilité de traction — de la zone fracturée des os. La consolidation osseuse paraît plus résistante quand une certaine contrainte de compression a été imposée progressivement à la zone en cours de réparation. Autrement dit, un meilleur développement osseux réparateur se produit quand la fracture est tenue moins rigidement en sens longitudinal et peut subir une certaine compression à partir d'une valeur initiale faible.

Le but principal de l'invention est d'apporter un dispositif de fixation des os fracturés qui conserve toujours la même rigidité en flexion et en traction et qui présente en translation dans le sens de la compression une rigidité réduite réglable autorisant des alternances de compression et d'absence de compression de la zone fracturée sous l'effet des mouvements de rééducation des articulations du membre concerné.

Un fixateur selon l'invention comprend au moins deux barres rigides en flexion servant chacune à la fixation de plusieurs broches ; chaque barre comprend un tube rigide divisé dans sa zone médiane en deux parties mises en prolongement coaxial l'une de l'autre et réunies par un moyen de guidage autorisant uniquement un déplacement relatif de ces deux parties entre une position initiale d'écartement et une position de rapprochement maximal seulement contre l'action d'un élément élastique, avec une amplitude prédéterminée. Le moyen de guidage contient l'élément élastique qui est disposé entre les deux parties en prolongement afin de s'opposer élastiquement à leur rapprochement.

De préférence, le moyen de guidage est un manchon et l'élément élastique est un bloc élastique de compression disposé à l'intérieur de ce manchon.

Des goupilles radiales traversent à la fois le manchon et le tube rigide de part et d'autre de l'élément élastique, les goupilles situées d'un côté de ce dernier passant à travers des trous allongés longitudinalement ménagés dans la partie correspondante du tube.

Selon un mode de réalisation préféré de l'invention, le tube et le manchon sont cylindriques, et des poussoirs à tête cylindrique et à tige filetée sont interposés entre chaque partie de tube et l'élément élastique, l'extrémité de ces parties étant filetée et les tiges des poussoirs y étant vissées, de sorte que l'on peut régler la longueur du logement réservé à l'élément élastique et, ainsi, imposer à ce dernier une précontrainte de compression.

De préférence, plusieurs trous allongés longitudinalement ayant des longueurs différentes sont prévus à travers au moins une des deux parties du tube pour recevoir une goupille transversale, permettant ainsi un réglage de la course de compression selon le trou allongé dans lequel est placée la goupille.

Dans une variante de l'invention, une entretoise de longueur fixe déterminée est placée dans le manchon, en série avec l'élément élastique entre les deux parties du tube, la longueur de cette entretoise déterminant la précontrainte de compression de l'élément élastique.

On donnera maintenant, uniquement à titre d'exemple, et sans exclure aucune variante, une description d'un mode préféré de réalisation de l'invention et de plusieurs variantes particulières qui peuvent lui être apportées. On se reportera aux dessins annexés dans lesquels :

la figure 1 est une vue d'une barre faisant partie d'un fixateur conforme à l'invention, représentée partiellement en coupe longitudinale,

la figure 2 est une vue de détail d'une partie de la barre de la figure 1,

la figure 3 est une vue en partie en coupe longitudinale analogue à la figure 1 montrant une première variante particulière de l'invention,

la figure 4 est une vue en coupe transversale selon IV-IV de la figure 3,

la figure 5 est une vue en partie en coupe longitudinale analogue à la figure 1 montrant une seconde variante particulière de l'invention,

la figure 6 est une vue générale montrant, pour fixer les idées, un cas d'utilisation du fixateur de l'invention,

la figure 7 est une vue analogue à la fig. 5 montrant une troisième variante de réalisation de l'invention.

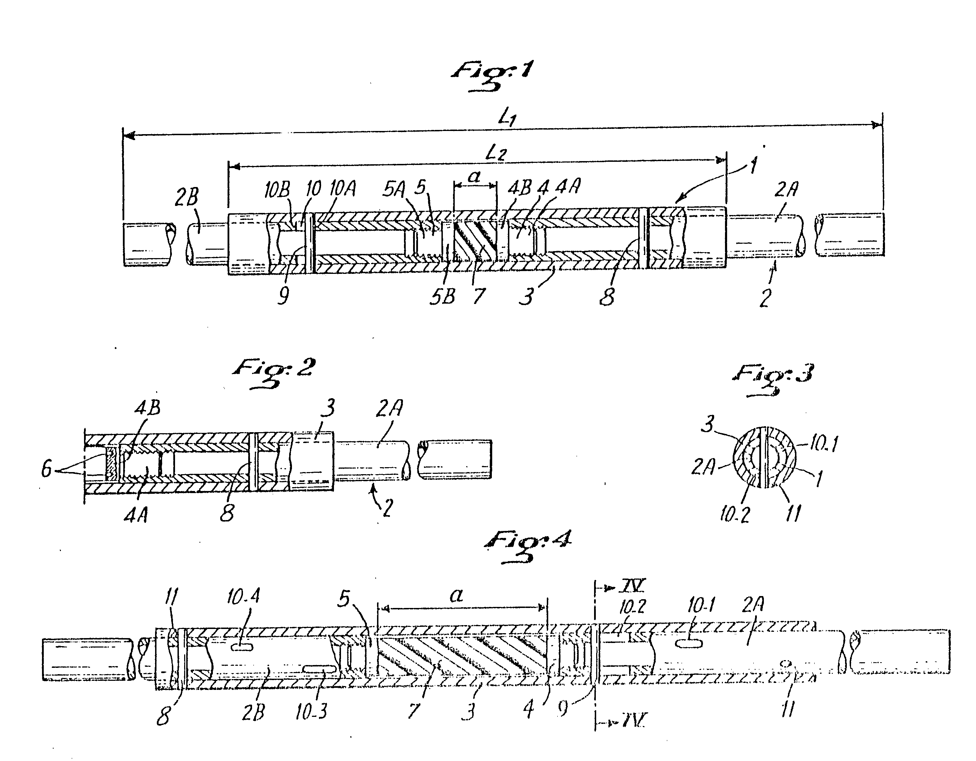

Un dispositif de fixation selon l'invention comprend au moins deux barres 1 qui sont placées dans deux plans ou dans plusieurs plans de fixation. Plusieurs broches sont associées à chaque barre 1 et sont vissées par une extrémité à travers un os brisé tel qu'un fémur, de part et d'autre d'une fracture de ce dernier. Toutes les broches situées d'un même côté d'une fracture sont tenues rigidement serrées par leur extrémité libre extérieure entre deux plaques et ces plaques sont elles-mêmes réunies rigidement par une barre. Ces moyens de fixation des broches et les broches elles-mêmes ne sont pas représentées sur les figures 1 à 5 parce que cet ensemble est de type classique et n'est pas concerné par l'invention.

Chaque barre 1 a une longueur $L_1$ qui peut être de 250 mm ou 300 mm ou plus, avec une zone médiane de longueur $L_2$ qui est de l'ordre de 120 à 150 mm. Cette zone médiane est celle qui se trouve sensiblement en regard de la fracture. Dans l'exemple illustré par la figure 1, la barre 1 comprend un tube rigide cylindrique 2 divisé en parties 2A, 2B qui sont mises en prolongement coaxial et tenues en cette position par un manchon 3. Ce dernier contient, sur une distance suffisante à assurer un bon maintien, une longueur de chaque partie 2A, 2B. En pratique le manchon 3 peut avoir la longueur $L_2$ indiquée plus haut comme étant celle de la zone médiane. Les parties 2A, 2B sont introduites avec un jeu très faible dans le manchon 3 (on peut adopter par exemple la tolérance d'ajustage H7g6) mais peuvent coulisser dans ce dernier.

A son extrémité intérieure au manchon 3, chaque partie 2A, 2B du tube 2 est filetée et un poussoir 4, 5, respectivement, est vissé par une tige filetée 4A, 5A à cette extrémité. Chaque poussoir 4, 5 a une tête cylindrique 4B, 5B de diamètre inférieur au diamètre intérieur du manchon 3. Il est utile de ménager dans chaque tête 4B, 5B deux trous borgnes longitudinaux espacés 6 qui permettent de visser et de dévisser commodément les poussoirs 4, 5 dans les extrémités des parties 2A, 2B avant l'introduction de celles-ci dans le manchon 3. Ce dernier contient aussi un bloc cylindrique 7 en élastomère qui est interposé et comprimé entre les têtes 4B, 5B des poussoirs 4, 5.

La partie 2A du tube 2 est immobilisée dans le manchon 3 grâce à une goupille transversale 8 emmanchée à force dans un trou diamétral percé à travers cette partie 2A et le manchon 3, d'un côté (le côté droit quand on regarde la figure 1) du bloc 7 en élastomère. Sur le côté opposé de ce dernier, une goupille transversale 9 est emmanchée à force dans un trou diamétral percé à travers le manchon 3. Cette goupille 9 passe dans un trou allongé longitudinalement 10 prévu dans les parois de la partie 2A du tube 2. Ce trou allongé 10 a une première extrémité 10A proche du bloc 7 en élastomère et une seconde extrémité 10B éloignée de ce bloc 7.

Sous l'effet du bloc 7 comprimé entre les poussoirs 4, 5 la partie 2B du tube 2 est poussée ; elle est retenue par l'extrémité 10A du trou allongé 10 qui rencontre la goupille 9. Si un effort de compression ou de rapprochement supérieur à la force de poussée du bloc élastique 7 est exercé sur les parties 2A, 2B, la partie 2B coulisse dans le manchon 3 jusqu'à ce que l'extrémité 10B du trou allongé 10 rencontre la goupille 9. Un tel déplacement se traduit par une compression exercée sur la zone osseuse de la fracture en cours de réparation. Le jeu des muscles pour mouvoir les articulations du membre blessé suffit à créer l'effort de rapprochement ou de compression qui surmonte celui du bloc élastique 7.

On peut régler à l'avance la force de rapprochement désirée en donnant au bloc 7 une précontrainte convenable de compression. Par exemple, quand on dévisse de plusieurs tours les poussoirs 4, 5 on diminue la longueur réservée au bloc élastique 7 et on accroît la valeur de la force de compression qui sera nécessaire à le comprimer davantage. On peut aussi modifier la nature ou la longueur à l'état libre du bloc élastique 7.

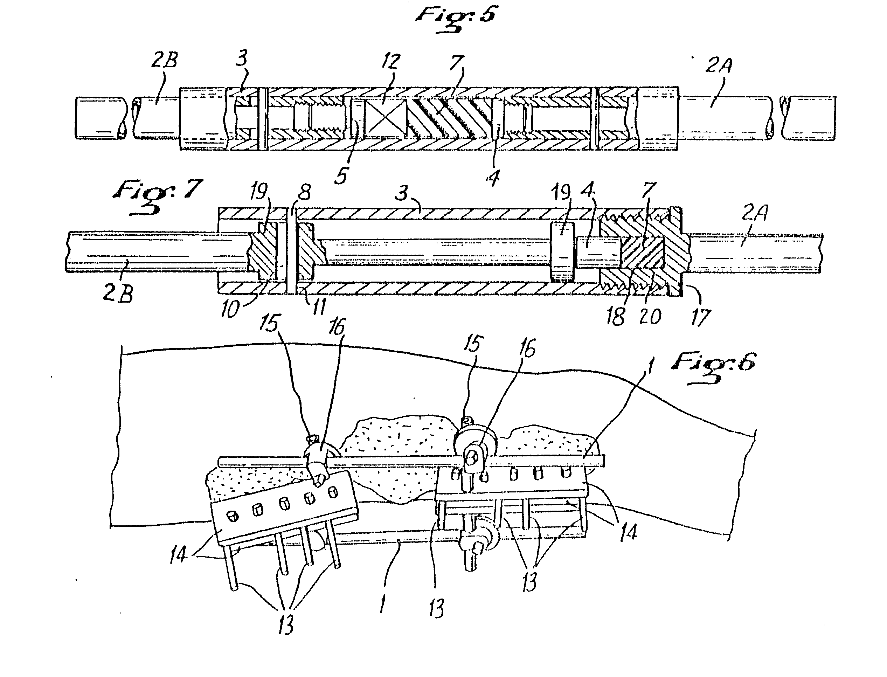

On peut, en plus, régler la course de compression ou de rapprochement des deux parties 2A, 2B du tube 2, comme le montre la figure 3. Selon cette variante de l'invention, il existe deux trous diamétraux 11 situés l'un d'un côté et l'autre de l'autre côté du bloc élastique 7, aptes chacun à recevoir une goupille 8 d'immobilisation. De chaque côté du bloc élastique 7 sont également prévus des trous allongés longitudinalement 10 à travers les parois des parties 2A et 2B, respectivement 10-1, 10-2 d'une part, 10-3, 10-4 d'autre part. A chacun de ces trous allongés correspond un trou transversal percé à travers le manchon 3. Ces trous 10-1 à 10-4 sont aptes à être traversés par une goupille transversale 9 ; ils ont des longueurs différentes, par exemple pour le trou 10-4 la longueur est de 3 mm, pour le trou 10-1 : 4,5 mm, pour le trou 10-3 : 6 mm, pour le trou 10-2 : 7,5 mm. Ils sont situés sur des génératrices différentes d'un même côté du bloc élastique 7, comme on peut le voir sur la figure 4. En correspondance avec les courses de compression autorisées par les trous allongés 10-1 à 10-4, on emploie des blocs élastiques 7 de longueur a croissante en même temps que celle de ces trous ; par exemple la longueur a est de 10 mm pour l'utilisation des trous 10-4, de 20 mm pour celle des trous 10-1, de 30 mm pour celle des trous 10-3, de 40 mm pour celle des trous 10-2.

De cette façon, il est possible pendant la durée de réparation d'une fracture de régler la course permise de compression, plus exactement de l'accroître progressivement en quatre fois en plaçant la goupille d'immobilisation 8 dans le trou radial 10 ou dans le trou radial 11 et en plaçant la goupille 9 successivement dans chacun des trous allongés 10-1 à 10-4. On peut à chaque fois modifier en correspondance la précontrainte de compression du bloc élastique 7 en agissant sur les poussoirs 4 et 5.

La figure 5 montre une autre manière de régler la précontrainte du bloc élastique 7. A l'intérieur du manchon 3, entre les parties 2A, 2B, plus exactement entre les poussoirs 4, 5 on dispose en série avec le bloc élastique 7 une entretoise rigide 12 dont la longueur est choisie pour compléter entre les poussoirs 4, 5 la longueur plus courte du bloc élastique 7. On peut, de cette façon changer dans de plus grandes proportions la longueur et la précontrainte de compression de ce dernier.

Pour fixer les idées, la figure 6 montre l'utilisation d'un fixateur conforme à l'invention pour la stabilisation d'une fracture d'un fémur. Quatre broches 13 sont vissées dans l'os d'un côté et de l'autre de la fracture. De chaque côté, les quatre broches sont dans un même plan ; ces deux plans ne sont pas nécessairement confondus. Chaque ensemble de quatre broches 13 est solidement serré entre deux plaques 14. Chacune de ces plaques 14 est munie perpendiculairement à son plan d'un barreau rigide 15. Un raccord double orientable 16 permet de réunir solidement deux barreaux 15 de part et d'autre de la fracture au moyen d'une barre 1 conforme à l'invention. On se sert nécessairement de deux barres l, au moins, par fracture. Pour effectuer les réglages expliqués plus haut, de la précontrainte du bloc élastique 7 et de la longueur permise de compression, on démonte une seule barre l, on la règle et on la remet en place dans les raccords 15 avant de démonter l'autre barre en vue de la régler à son tour.

Selon la variante illustrée par la figure 7, la partie 2A est terminée du côté du manchon 3 par un élargissement extrême 20 fileté extérieurement suivi d'une collerette d'arrêt 17 à plus grand diamètre. L'extrémité correspondante du manchon 3 est filetée intérieurement ; ainsi cette partie 2A est immobilisée par rapport au manchon 3 par vissage jusqu'à appui de ce dernier contre la collerette 17.

L'élargissement 20 a un logement borgne 18, de préférence coaxial au manchon 3, s'ouvrant sur la face extrême terminale de l'élargissement 20, de profondeur supérieure à la longueur d'un bloc élastique 7 qui est introduit dans ce logement 18. Ce dernier contient encore un poussoir 4 qui est appuyé contre le bloc élastique 7 et qui est saillant hors de la pièce 2A, à l'intérieur du manchon 3.

La partie 2B a un diamètre extérieur de préférence égal à celui de la partie 2A, exceptés la collerette 17 et l'élargissement 20. Ce diamètre est inférieur au diamètre intérieur du manchon 3. Pour être guidée dans ce dernier, la partie 2B a deux zones espacées 19 de longueur restreinte, suffisante au guidage, de diamètre accru à la dimension voulue pour coulisser à l'intérieur du manchon 3. Dans une zone 19 est ménagée une ouverture 10 allongée longitudinalement et contenant la goupille 8 qui traverse les trous diamétraux 11 percés dans le manchon 3. La partie 2A s'appuie contre le poussoir 4 pour l'écrasement du bloc élastique 7, comme expliqué plus haut. Bien qu'il soit abrité dans le logement 18, ce bloc élastique 7 est toujours contenu dans le manchon 3 qui guide les parties 2A, 2B.

## Revendications

1. Dispositif de fixation externe pour immobilisation de broches utilisées en chirurgie orthopédique comprenant au moins deux barres (1) rigides en flexion, chaque barre (1) comprenant deux parties (2A, 2B) mises en prolongement coaxial et déplaçables relativement avec interposition d'un élément élastique (7), caractérisé en ce que ce dispositif comprend un moyen de guidage (3) contenant une partie extrême de chacune des deux parties (2A, 2B), ce moyen de guidage (3) autorisant leur déplacement relatif en translation avec une amplitude totale prédéterminée entre une position initiale d'écartement et une position de rapprochement maximal seulement, l'élément élastique (7) étant contenu dans le moyen de guidage (3) et disposé pour s'opposer élastiquement à ce rapprochement.

2. Dispositif selon la revendication 1 caractérisé en ce que le moyen de guidage (3) immobilise les parties (2A, 2B) en rotation l'une par rapport à l'autre.

3. Dispositif selon la revendication 1 caractérisé en ce que le moyen de guidage est un manchon (3) dans lequel une partie extrême d'une partie (2B) est guidée en translation tandis que la partie extrême de l'autre partie (2A) est fixée de manière démontable à ce manchon.

4. Dispositif selon la revendication 3 caractérisé en ce que l'élément élastique est un bloc élastique de compression (7) disposé à l'intérieur du manchon (3) entre les deux parties (2A, 2B) mises en prolongement.

5. Dispositif selon l'une quelconque des revendications 1 à 4 caractérisé en ce que l'élément élastique (7) est en état de précontrainte de compression.

6. Dispositif selon la revendication 5 caractérisé en ce qu'il comprend des moyens de réglage de la précontrainte de compression du bloc élastique (7).

7. Dispositif selon la revendication 1 dans lequel les parties (2A, 2B) sont cylindriques, caractérisé en ce que le manchon (3) est également cylindrique, les parties extrêmes des parties (2A, 2B) qui y sont contenues sont filetées tandis que des poussoirs (4, 5) ayant une tige filetée (4A, 5A) et une tête (4B, 5B) sont vissés dans les parties (2A, 2B) et contiennent entre leurs têtes un bloc élastique (7) en état de précontrainte de compression.

8. Dispositif selon la revendication 2 caractérisé en ce que le déplacement relatif des deux parties (2A, 2B) est rendu possible par une broche transversale (9) engagée dans un trou transversal percé à travers le moyen de guidage (3) et dans un trou (10) allongé longitudinalement ménagé à travers une partie (2A, 2B) du tube (2) tandis que l'autre partie (2B ou 2A) est réunie de manière fixe au moyen de guidage (3).

9. Dispositif selon la revendication 8 caractérisé en ce qu'il existe sur le moyen de guidage (3) plusieurs trous transversaux en correspondance avec plusieurs trous (10) allongés longitudinalement ayant des longueurs différentes progressivement croissantes, ménagés dans l'une au moins des deux parties (2A, 2B).

10. Dispositif selon l'une quelconque des revendications 3 et 6 caractérisé en ce qu'une entretoise rigide (12) est montée en série avec le bloc élastique (7) à l'intérieur du moyen de guidage (3), entre les deux parties (2A, 2B).

11. Dispositif selon la revendication 8 caractérisé en ce que l'autre partie (2A ou 2B) est réunie au manchon (3) par vissage dans celui-ci d'un élargissement extrême (20) fileté extérieurement suivi d'une collerette d'arrêt (17), cet élargissement (20) ayant un logement borgne (18) de longueur supérieure à celle de l'élément élastique (7) afin de contenir celui-ci et un poussoir (4) saillant hors de ce logement (18) à l'intérieur du manchon (3).

**Claims**

1. A device for external attachment for immobilizing pins employed in orthopaedic surgery, comprising at least two bars (1) which are rigid against bending and each bar (1) of which comprises two portions (2A, 2B) placed in coaxial prolongation and able to be displaced relatively with the interposition of a resilient member (7), characterised in that this device includes a means of guidance (3) containing one end portion of each of the two portions (2A, 2B), this means of guidance (3) allowing their relative displacement in translation with a predetermined total amplitude between an initial position of separation and a position of maximum approach only, the resilient member (7) being contained in the means of guidance (3) and arranged in order to be opposed resiliently to this approach.

2. A device as in Claim 1, characterised in that the means of guidance (3) immobilizes the portions (2A, 2B) in rotation with respect to one another.

3. A device as in Claim 1, characterised in that the means of guidance is a sleeve (3) in which one end portion of one portion (2B) is guided in translation whilst the end portion of the other portion (2A) is fixed to this sleeve dismountably.

4. A device as in Claim 3, characterised in that the resilient member is a resilient compression block (7) arranged inside the sleeve (3) between the two portions (2A, 2B) placed in prolongation.

5. A device as in any one of the Claims 1 to 4, characterised in that the resilient member (7) is in a state of compressive prestress.

6. A device as in Claim 5, characterised in that it includes means of regulation of the compressive prestress in the resilient block (7).

7. A device as in Claim 1 in which the portions (2A, 2B) are cylindrical, characterised in that the sleeve (3) is likewise cylindrical, the end portions of the portions (2A, 2B) which are contained in it are threaded whilst thrusters (4, 5) having a threaded rod (4A, 5A) and a head (4B, 5B) are screwed into the portions (2A, 2B) and contain between their heads a resilient block (7) in a state of compressive prestress.

8. A device as in Claim 2, characterised in that the relative displacement of the two portions (2A, 2B) is made possible by a transverse pin (9) engaging in a transverse hole drilled through the means of guidance (3) and in a hole (10) elongated longitudinally, arranged through one portion (2A, 2B) of the tube (2) whilst the other portion (2B or 2A) is joined in a fixed manner to the means of guidance (3).

9. A device as in Claim 8, characterised in that there exists in the means of guidance (3) a number of transverse holes corresponding with a number of holes (10) elongated longitudinally and having different progressively increasing lengths, arranged in at least one of the two portions (2A, 2B).

10. A device as in either of the Claims 3 and 6, characterised in that a rigid spacer (12) is mounted in series with the resilient block (7) inside the means of guidance (3) between the two portions (2A, 2B).

11. A device as in Claim 8, characterised in that the other portion (2A or 2B) is joined to the sleeve (3) by screwing into the latter an end enlargement (20) having an external thread followed by a stop-collar (17), this enlargement (20) having a blind seating (18) of a length greater than that of the resilient member (7) in order to contain the latter and a thruster (4) projecting out of this seating (18) into the interior of the sleeve (3).

**Patentansprüche**

1. Vorrichtung zum Aussenfixieren zwecks Immobilisierung von Spindeln, die bei der orthopädischen Chirurgie verwendet werden, mit mindestens zwei bezüglich einer Biegung starren Stäben (1), wovon jeder zwei Teile (2A, 2B) umfasst, die in koaxialer Verlängerung zueinander angeordnet und relativ zueinander unter Zwischenschaltung eines Elastikelements (7) verschiebbar sind, dadurch gekennzeichnet, dass die Vorrichtung eine Führungseinrichtung (3) aufweist, die einen Endabschnitt eines jeden der beiden Teile (2A, 2B) aufnimmt, und die Führungseinrichtung (3) ihre relative Translationsbewegung mit einer vorgegebenen Gesamtamplitude zwischen einer anfänglichen Abstandsstellung und allein einer maximalen Annäherungsstellung gestattet, wobei das Elastikelement (7) in der Führungseinrichtung (3) enthalten und derart angeordnet ist, um sich elastisch dieser Annäherung zu widersetzen.

2. Vorrichtung nach Anspruch 1, dadurch gekennzeichnet, dass die Führungseinrichtung (3) die beiden Teile (2A, 2B) an einer Drehung relativ zueinander hindert.

3. Vorrichtung nach Anspruch 1, dadurch gekennzeichnet, dass die Führungseinrichtung eine Hülse (3) ist, in welcher ein Endabschnitt des einen Teils (2B) bezüglich einer Translationsbewegung geführt wird, während der andere Endabschnitt des anderen Teils (2A) abnehmbar an der Hülse befestigt ist.

4. Vorrichtung nach Anspruch 3, dadurch gekennzeichnet, dass das Elastikelement ein elastischer Druckblock (7) ist, der im Inneren der Hülse (3) in Verlängerung zwischen den beiden Teilen (2A, 2B) angeordnet ist.

5. Vorrichtung nach einem der Ansprüche 1 bis 4, dadurch gekennzeichnet, dass sich das Elastikelement im Zustand einer Druckvorspannung befindet.

6. Vorrichtung nach Anspruch 5, dadurch gekennzeichnet, dass sie eine Einstellvorrichtung für die Druckvorspannung des elastischen Blocks (7) aufweist.

7. Vorrichtung nach Anspruch 1, in welcher die Teile (2A, 2B) zylindrisch sind, dadurch gekennzeichnet, dass die Hülse (3) ebenfalls zylindrisch ist, und dass die Endabschnitte der Teile (2A, 2B), die darin untergebracht sind, ein Gewinde aufweisen, während Druckstücke (4, 5) mit einem Gewindestab (4A, 5A) und einem Kopf (4B, 5B) in die Teile (2A, 2B) eingeschraubt sind und zwischen ihren Köpfen einen Elastikblock (7) im Zustand der Druckvorspannung aufweisen.

8. Vorrichtung nach Anspruch 2, dadurch gekennzeichnet, dass die Relativverschiebung der beiden Teile (2A, 2B) durch eine Querspindel (9) ermöglicht wird, die in ein Querloch eingreift, welches die Führungseinrichtung (3) durchdringt, sowie in ein Langloch (10), das sich über einen Teil (2A, 2B) des Rohres (2) erstreckt, während der andere Teil (2B oder 2A) fest mit der Führungseinrichtung (3) verbunden ist.

9. Vorrichtung nach Anspruch 8, dadurch gekennzeichnet, dass auf der Führungseinrichtung (3) mehrere Querlöcher vorhanden sind, die mit mehreren Langlöchern (10) zusammenwirken, welche unterschiedliche und zunehmend grössere Längen haben und mindestens in einem der beiden Teile (2A, 2B) angeordnet sind.

10. Vorrichtung nach einem der Ansprüche 3 und 6, dadurch gekennzeichnet, dass ein starres Zwischenstück (12) in Reihe mit dem Elastikblock (7) im Inneren der Führungseinrichtung (3) zwischen den beiden Teilen (2A, 2B) liegt.

11. Vorrichtung nach Anspruch 8, dadurch gekennzeichnet, dass der andere Teil (2A oder 2B) mit der Hülse (3) verbunden ist, indem in diese eine endseitige, mit Gewinde versehene Erweiterung eingeschraubt ist, an die sich aussen ein Haltekragen (17) anschliesst, wobei die Erweiterung (20) eine einseitige Ausnehmung (18) aufweist, deren Länge grösser als jene des Elastikelements (7) ist, um dieses und ein Druckstück (4) aufzunehmen, welches über die Ausnehmung (18) in das Innere der Hülse (3) hineinragt.

*Fig:1*

$L_1$

$L_2$

2B    10B 10 10A    5A 5    $a$    4B 4 4A    1    2A

9    5B 7    3    8    2

*Fig:2*

4B    3    2A

6    4A    8    2

*Fig:3*

3    10.1

2A    1

10.2    11

*Fig:4*

11    10.4    5    $a$    IV    10.2    10.1    2A

8    2B    10.3    7    3    4    9    IV    11

Fig.5

Fig.7

Fig.6